# EUROPEAN PATENT APPLICATION

(11) **EP 3 123 929 A1**
(43) Date of publication of application: **01.02.2017**
(21) Application number: 15178646.4
(22) Date of filing: 28.07.2015
(51) Int. Cl.: A61B 5/00, A41D 13/12, A61B 5/0205, A61B 5/11

(54) **FABRIC INCLUDING DETECTION MODULE**

(71) Applicant: King's Metal Fiber Technologies Co., Ltd., 42060 Taichung City (TW)
(72) Inventor: WANG, Hao Chen, Taipei City 10451 (TW); CHANG, Li Chuan, Taipei City 10451 (TW); LIAO, Shu Fen, Taipei City 10451 (TW); KANG, Yu Hsun, Taipei City 10451 (TW); CHEN, Reng Sho, Taipei City 10451 (TW)
(74) Representative: Cabinet Chaillot

(57) **Abstract**

A detection module-included fabric includes an arranged combination of at least one detection module, a surface layer, and a fabric layer. The detection module includes a contact surface. The surface layer is combined with the at least one detection module. The detection module is below the surface layer. The surface layer includes at least one window opening. The contact surface of the detection module is exposed through the window opening of the surface layer. The fabric layer is combined with the surface layer. A gap is formed between the fabric layer and the surface layer. The detection module is located between the surface layer and the fabric layer. As such, the detection module mounted to the surface layer is hard to slide or shift in position so that the detection module is constantly kept in contact engagement with a surface of a human body for stabilization of signal detection.

## Description

### BACKGROUND OF THE INVENTION µ

### 1. Field of the Invention

The present invention relates to a fabric including a detection module, and more particularly to one that comprises an arranged combination of at least one detection module, a surface layer, and a fabric layer to allow the detection module to keep constant contact engagement with a surface of a human body in order to improve stability of signal detection and is applicable to a physiological detective garments, an inclination detective garment, or the likes.

### 2. Description of Related Art

The progress of science and technology makes it possible to develop a combination of inspection equipment with clothing in order to facilitate inspect and record the physiological conditions of a human body and allows for applications to self-management of home healthcare or preventive medical treatments.

Heretofore, the inspection equipment is arranged on clothing that is closely adjacent to a surface of a skin in order to allow the inspection equipment to engage the skin surface for detection of physiological signals of the human body. However, the skin surface comprises pores, which are provided for heat dissipation and regulation of body temperature.

If the clothing that is closely adjacent to the skin surface is clothing that is tightly fit to the human body, unless it has excellent water diffusion and wicking property, it would become uncomfortable after long time wearing and even intolerable, making it hard for detection of physiological signal with the inspection equipment for an extended period of time.

If a garment that is large is size and thus loosely fit to the human body, the garment would be spaced from the skin surface by a distance so that during the detection of the physiological signals with the inspection equipment, a movement of the human body would cause the garment to make a relative slide between the inspection equipment and the location of the skin surface where the equipment is originally set. Consequently, the equipment cannot be kept in contact with the skin surface, leading to an undesired situation of intermittent detection with the detection signal being irregularly interrupted. This is quite troublesome to those that intent to make measurements for self-management of home healthcare or preventive medical treatments. In addition, the data of the detection may be unexpectedly distorted, making it not possible to present an actual condition.

Thus, in view of the above problems, the present invention aims to provide a fabric including a detection module that prevents undesired positional shift and allows a detection module to be kept in constant contact engagement with a surface of a human body and features easy operation and installation.

### SUMMARY OF THE INVENTION

The primary objective of the present invention is to provide a detection module-included fabric, which comprises an arranged combination of at least one detection module, a surface layer, and a fabric layer. The detection module includes a contact surface. The surface layer is combined with the at least one detection module and the detection module is below the surface layer. The surface layer includes at least one window opening and the contact surface of the detection module is exposed through the window opening of the surface layer. The fabric layer is combined with the surface layer and a gap is formed between the fabric layer and the surface layer. The detection module is located between the surface layer and the fabric layer. As such, with the contact surface of the detection module that is exposed through the window opening of the surface layer being positionable against and contactable with the surface of the human body, when the fabric layer is shifted due to a movement of the user, since the contact surface of the detection module is positionable against the surface of the human body and shows an increased frictional force therebetween, during stretching or moving of the fabric layer, the contact surface of the detection module exhibits excellent attachment to the surface of the human body so that the contact engagement thereof with the surface of the human body is enhanced to make it hard for the detection module that is mounted to the surface layer to slide and thus, the detection module is constantly kept in contact engagement with the surface of the human body to improve stability of signal detection and also improve the utilization of the device.

A secondary objective of the present invention is to provide a detection module-included fabric, in which the fabric layer has an edge that comprises at least one sewing thread. The sewing thread of the fabric layer is combined with an edge of the surface layer. The combination is achieved with one of sewing, ultrasonic fusion, hot fusion, and adhesive. With the contact surface of the detection module that is exposed through the window opening of the surface layer being positionable against and contactable with the surface of the human body, when the fabric layer is shifted due to a movement of the user, since the contact surface of the detection module is positionable against the surface of the human body and shows an increased frictional force therebetween, during stretching or moving of the fabric layer, the contact surface of the detection module exhibits excellent attachment to the surface of the human body so that the contact engagement thereof with the surface of the human body is enhanced thereby improving the overall attachment of the device.

A further objective of the present invention is to provide a detection module-included fabric, in which the detection module is located between the surface layer and the fabric layer and the contact surface of the detection module is exposed through the window opening of the surface layer to be set in constant contact engagement with a surface of a human body. The detection module comprises an electrode plate that detects a physiological signal of the surface layer of the human body. The physiological signal can be one of body temperature, heartbeat, and pulse. Further, the detection module may alternatively comprise an inclination detection chip and a microcontroller. The microcontroller is connected to the inclination detection chip and the microcontroller detects a variation of the inclination detection chip. Thus, in addition to detection of a variation of the physiological signal of the body, the detection module also detects a variation of an inclination angle of the human body in order to identify an event of dizziness or fall thereby improving overall safety.

To achieve the above objectives, the present invention provides a detection module-included fabric, which comprises: at least one detection module, a surface layer, and a fabric layer. The detection module comprises a contact surface. The surface layer is combined with the at least one detection module. The detection module is located below the surface layer. The surface layer comprises at least one window opening and the contact surface of the detection module is exposed through the window opening of the surface layer. The fabric layer is combined with the surface layer and a gap is formed between the fabric layer and the surface layer. The detection module is located between the surface layer and the fabric layer.

### BRIEF DESCRIPTION OF THE DRAWINGS

The present invention can be fully understood from the following detailed description and preferred embodiment with reference to the accompanying drawings, in which:
Figure 1 is a perspective view shows a first embodiment of the present invention;
Figure 2 is an exploded view of the first embodiment of the present invention;
Figure 3 is a partial enlarged view of the first embodiment of the present invention;
Figure 4 is a cross-sectional view of the first embodiment of the present invention taken along line A-A;
Figure 5 is a schematic view illustrating another example of the site where the first embodiment of the present invention is embodied;
Figure 6 is a perspective view shows a second embodiment of the present invention;
Figure 7 is an exploded view of the second embodiment of the present invention;
Figure 8 is a partial enlarged view of the second embodiment of the present invention; and
Figure 9 is a cross-sectional view of the second embodiment of the present invention taken along line A-A.

### DETAILED DESCRIPTION OF THE PREFERRED EMBODIMENTS

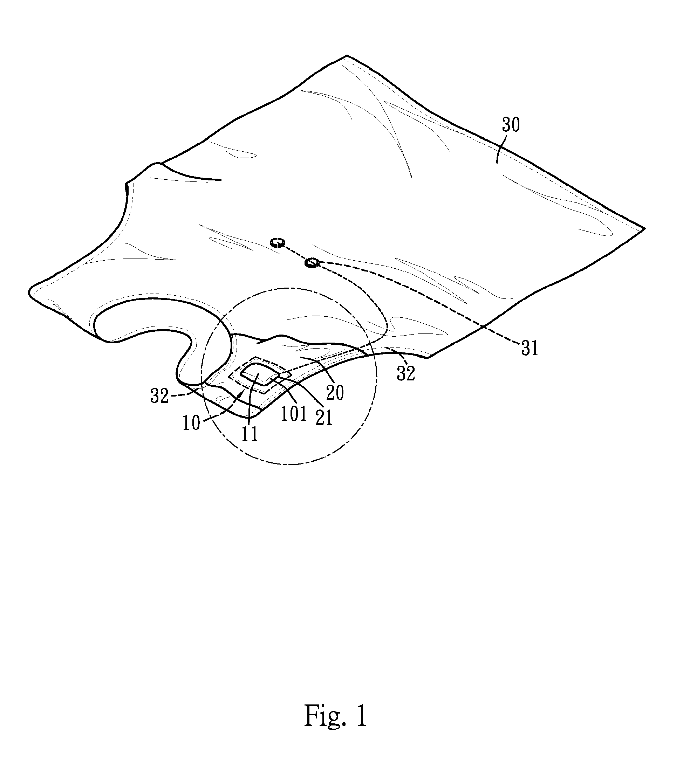

Referring to Figures 1-9, which are views illustrating embodiments of the present invention, a preferred embodiment of a fabric including a detection module according to the present invention is applicable to detection of variation of a physiological signal of a surface layer of a human body or an inclination angle of a human body and also for prevention of slip of the detection module 10 to constantly maintain contact engagement with a surface of the human body to thereby improve stability of signal detection.

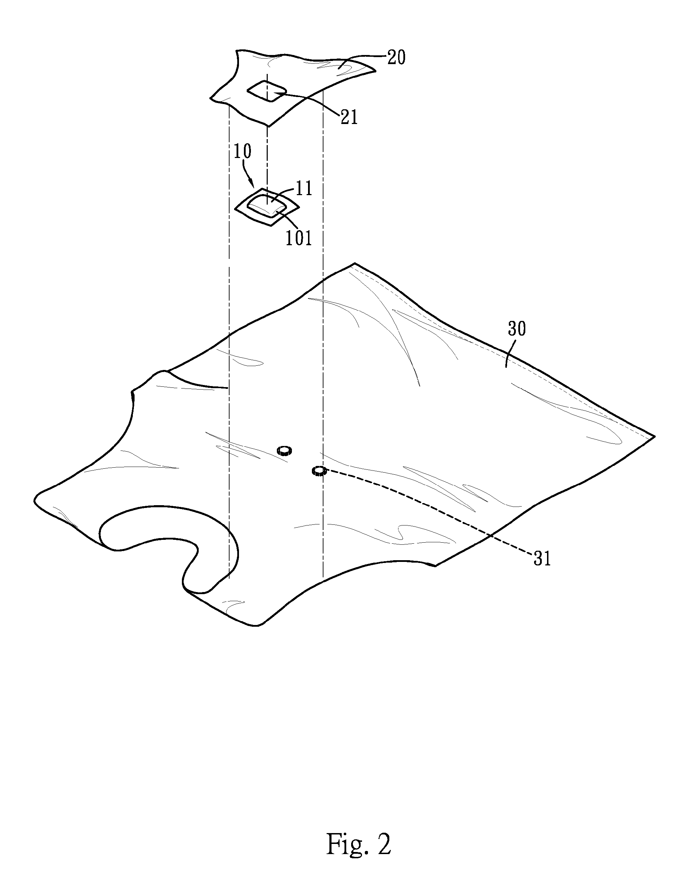

A first embodiment of the detection module-included fabric according to the present invention (as shown in Figures 1-4) comprises at least one detection module 10, a surface layer 20, and a fabric layer 30 (as shown in Figure 2). The detection module 10 comprises a contact surface 11 and the contact surface 11 of the detection module 10 is positionable on and contactable with the surface of the human body in order to detect the physiological signal of the surface layer of the human body. The detection module 10 is located below the surface layer 20. The surface layer 20 can be one of a piece of fabric, a woven article, and a membrane. (In the instant embodiment, a piece of fabric is used as an example for the present invention.) The membrane can be a plastic membrane, a metal membrane, a membrane of a carbon material, or a membrane of any other suitable materials. The detection module 10 is combined with the surface layer 20 and the combination can be achieved with one of sewing, ultrasonic fusion, hot fusion, and adhesive, or any other suitable ways of combination. (In the instant embodiment, sewing or stitching is used as example for illustration of the present invention.)

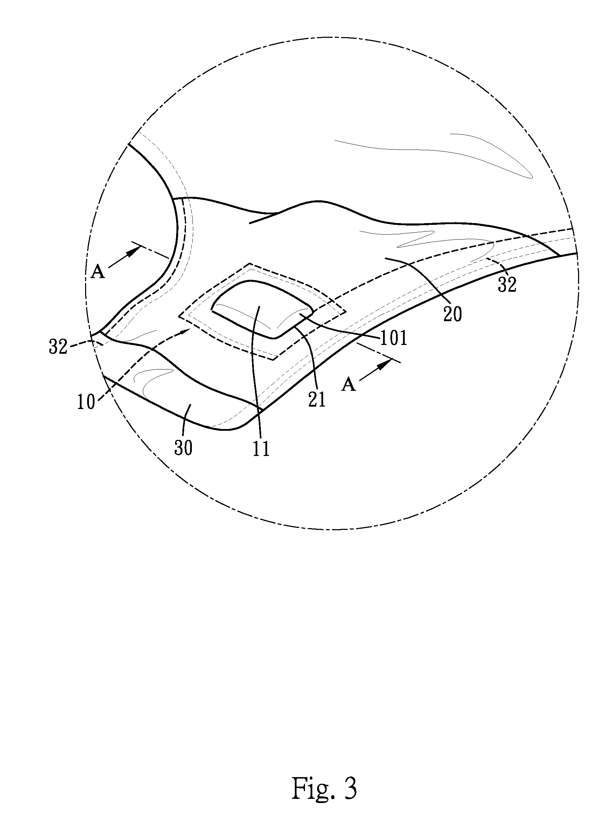

Further, the surface layer 20 comprises at least one window opening 21 formed therein. The number of the window opening 21 used is in consistent with that of the detection module 10. Thus, for example, when two detection modules 10 are included, the surface layer 20 is provided with two window openings 21 respectively corresponding thereto; or alternatively, each of the two detection modules 10 comprises a surface layer 20 corresponding thereto and the surface layer 20 comprises a window opening 21 corresponding thereto. Further, the contact surface 11 of the detection module 10 is exposed through the window opening 21 of the surface layer 20 (as shown in Figure 3), so that the contact surface 11 of the detection module 10 that is exposed through the window opening 21 of the surface layer 20 is attachable to the surface of the human body and can be kept in contact engagement therewith to provide stability of signal detection.

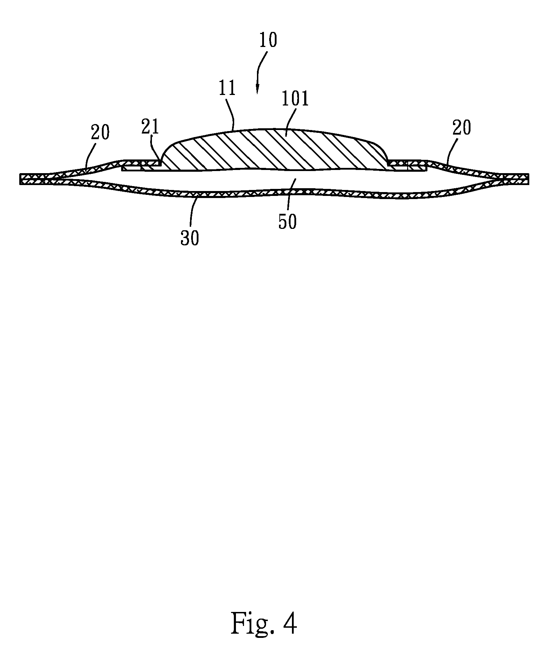

Further, the surface layer 20 is combinable with the fabric layer 30 and the combination can be one of sewing, ultrasonic fusion, hot fusion, and adhesive, or any other suitable ways of combination. (In the instant embodiment, sewing or stitching is used as example for illustration of the present invention.). The combination is such that a gap 50 is formed between the fabric layer 30 and the surface layer 20 so that the detection module 10 is located between the surface layer 20 and the fabric layer 30 (as shown in Figure 4). Further, in an embodiment of the present invention, the fabric layer 30 can be a portion of or tailored and stitched as a vest (or other types of clothing, such as a garment, trousers, gloves, an underwear, a corsage, and a tube-top, or the likes), so that the vest so made of the fabric layer 30 is wearable on a human body and in contact with a surface of the human body (the vest so made of the fabric layer 30 being shown inside out in the drawings) and the fabric layer 30 that is tailored and stitched or that is formed as a portion of the vest is put in tight engagement with a specific portion of the surface of the human body, such as the chest or the pit of the stomach.

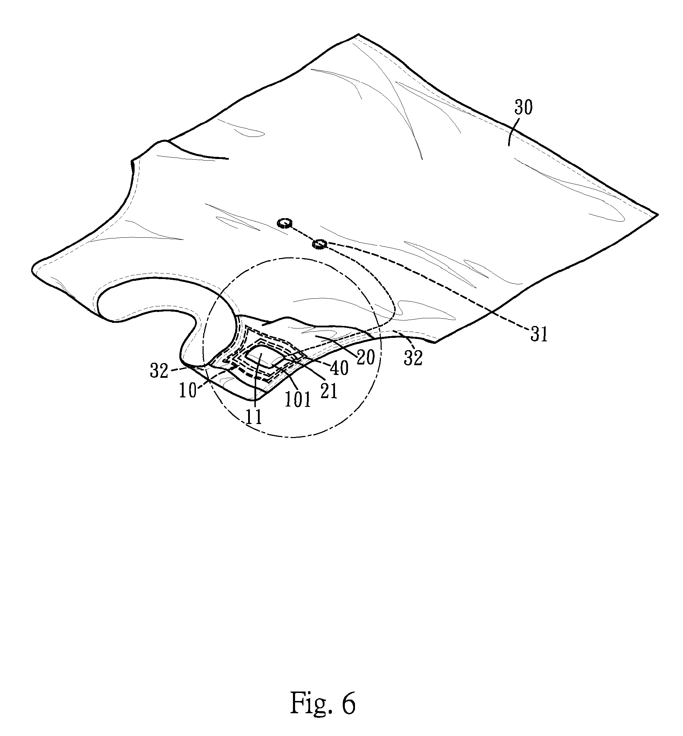

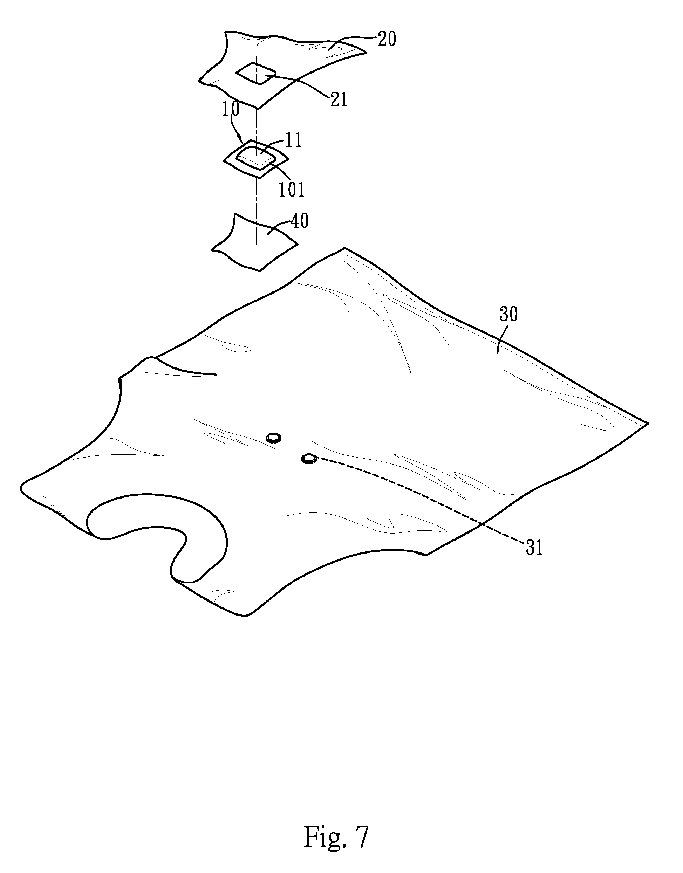

A second embodiment of the detection module-included fabric according to the present invention (as shown in Figures 6-9) comprises at least one detection module 10, a surface layer 20, a fabric layer 30, and a protection layer 40 (as shown in Figure 7). The protection layer 40 is arranged between the detection module 10 and the fabric layer 30 and a circumferential edge of the protection layer 40 is combined with the surface layer 20 and the combination can be achieved with one of sewing, ultrasonic fusion, hot fusion, and adhesive, or any other suitable ways of combination. (In the instant embodiment, sewing or stitching is used as example for illustration of the present invention.) The protection layer 40 can be a piece of fabric, an insulation sheet, or any other object that provides an effect of isolation. (In the instant embodiment, a piece of fabric is used as example for illustration of the present invention.) Thus, the protection layer 40 effectively isolates any engagement with one side of the detection module 10.

Further, the detection module 10 of the present invention can be combined with the protection layer 40 and the detection module 10 is combined with the protection layer 40 in such a way that the circumferential edge of the protection layer 40 comprises an extension flange (not shown) and the extension flange of the protection layer 40 is combined to the surface layer 20. The combination can be achieved with one of sewing, ultrasonic fusion, hot fusion, and adhesive, or any other ways of combination. If the protection layer 40 is formed of a piece of fabric of which an outer edge can be attached to the surface layer 30 through sewing. In addition, the protection layer 40 that is formed of a piece of fabric features softness, helping reduce discomfort. If the protection layer 40 (not shown) is formed of an insulation sheet of which an outer edge can be attached to the surface layer through ultrasonic fusion. In addition, the protection layer 40 that is formed of the insulation sheet features protection against damage caused by electrostatic charges resulting from rubbing.

The detection module 10 comprises a contact surface 11. The contact surface 11 of the detection module 10 is positionable on and contactable with the surface of the human body to detect the physiological signal of the surface layer of the human body. The detection module 10 is located below the surface layer 20. The surface layer 20 can be one of a piece of fabric, a woven article, and a membrane. (In the instant embodiment, a piece of fabric is used as an example for the present invention.) The membrane can be a plastic membrane, a metal membrane, a membrane of a carbon material, or a membrane of any other suitable materials. The detection module 10 is combined with the surface layer 20 and the combination can be achieved with one of sewing, ultrasonic fusion, hot fusion, and adhesive, or any other suitable ways of combination. (In the instant embodiment, sewing or stitching is used as example for illustration of the present invention.)

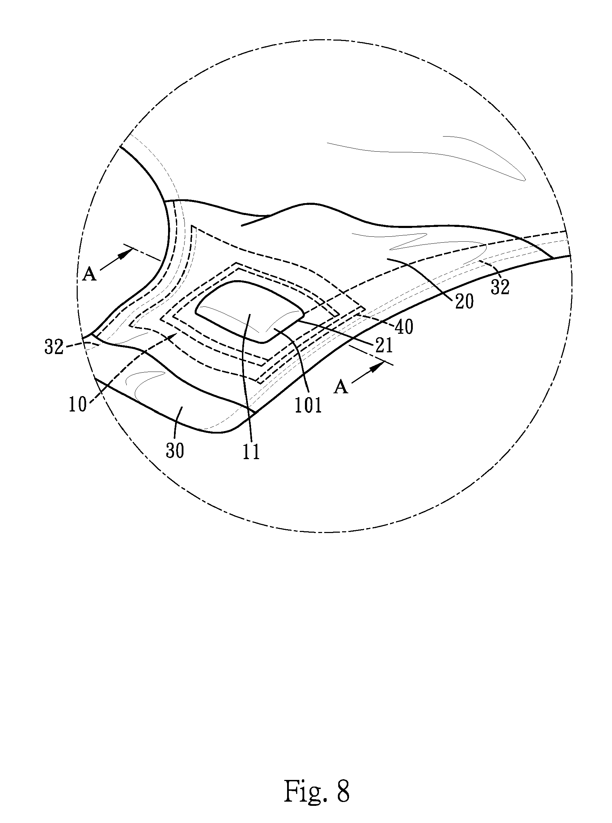

Further, the surface layer 20 comprises at least one window opening 21 formed therein. The number of the window opening 21 used is in consistent with that of the detection module 10. Thus, for example, when two detection modules 10 are included, the surface layer 20 is provided with two window openings 21 respectively corresponding thereto; or alternatively, each of the two detection modules 10 comprises a surface layer 20 corresponding thereto and the surface layer 20 comprises a window opening 21 corresponding thereto. Further, the contact surface 11 of the detection module 10 is exposed through the window opening 21 of the surface layer 20 (as shown in Figure 8), so that the contact surface 11 of the detection module 10 that is exposed through the window opening 21 of the surface layer 20 is attachable to the surface of the human body and can be kept in contact engagement therewith to provide stability of signal detection.

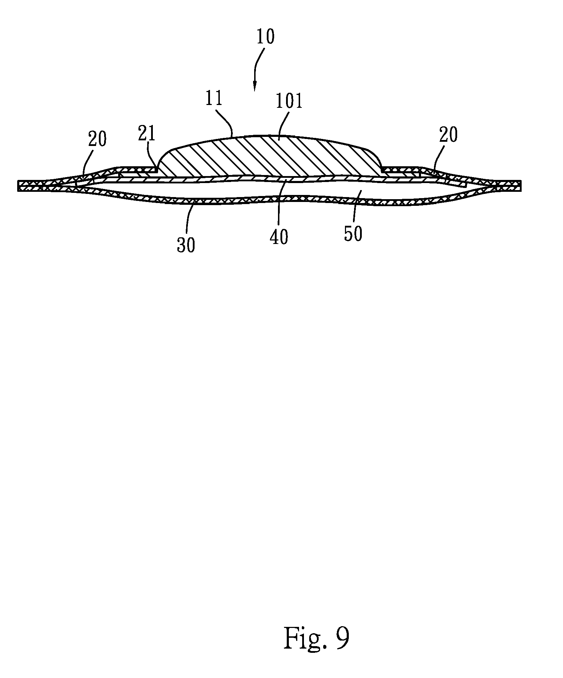

Further, the surface layer 20 is combinable with the fabric layer 30 and the combination can be one of sewing, ultrasonic fusion, hot fusion, and adhesive, or any other suitable ways of combination. (In the instant embodiment, sewing or stitching is used as example for illustration of the present invention.). The combination is such that a gap 50 is formed between the fabric layer 30 and the surface layer 20 so that the detection module 10 is located between the surface layer 20 and the fabric layer 30 (as shown in Figure 9). Further, in an embodiment of the present invention, the fabric layer 30 can be a portion of or tailored and stitched as a vest (or other types of clothing, such as a garment, trousers, gloves, an underwear, a corsage, and a tube-top, or the likes), so that the vest so made of the fabric layer 30 is wearable on a human body and in contact with a surface of the human body (the vest so made of the fabric layer 30 being shown inside out in the drawings) and the fabric layer 30 that is tailored and stitched or that is formed as a portion of the vest is put in tight engagement with a specific portion of the surface of the human body, such as the chest or the pit of the stomach.

A first illustrative embodiment and a second illustrative embodiment of the detection module 10 of the detection module-included fabric according to the present invention are provided for demonstration. The first illustrative embodiment comprises an electrode plate 101 (as shown in Figures 1 and 6) that detects the physiological signal of the surface layer of the human body. The electrode plate 101 is formed by weaving, knitting, or otherwise combining a plurality of non-conductive fiber yarns and a plurality of conductive fiber yarns; or alternatively, the electrode plate 101, in the entirety thereof, is formed solely of a plurality of conductive fiber yarns through weaving, knitting, or other combining means so as to make the entirety of the electrode plate 101 electrically conductive. Further, the plurality of conductive fiber yarns of the electrode plate 101 is woven, knitted, or otherwise combined to form a conductive zone. The conductive zone of the electrode plate 101 is positionable against and thus in tight contact engagement with the skin of the human body in order to detect the physiological signal. The physiological signal can be one of body temperature, heartbeat, and pulse.

For the first illustrative embodiment and the second illustrative embodiment of the detection module 10 of the detection module-included fabric according to the present invention that are provided for demonstration, the second illustrative embodiment comprises an inclination detection chip and a microcontroller (not shown). The microcontroller is connected to the inclination detection chip so that the microcontroller may detect a variation of the inclination detection chip. The inclination detection chip can be a three-axis acceleration transducer (such as a three-axis low-g micro-machined accelerometer). The three-axis acceleration transducer is operable to detect/calculate inclination angles and accelerations in three axes of X, Y, and Z and the microcontroller is operable to periodically detect and transmit these values. The inclination angle can be used to detect an event of dizziness or fall by setting a change exceeding a threshold of +/-1.0 degree, +/-1.5 degrees, or +/-2.0 degrees. The microcontroller can be set to conduct detection at a fixed time interval with a fixed number of detection operations. For example, abnormal inclination may be identified by means of an average of successively detected values within a period of 30 seconds being determined exceeding +/-1.0 degree (or +/-1.5 degrees or +/-2.0 degrees). Or alternatively, a nine-axis body position transducer may be used. The nine-axis body position transducer comprises a three-axis acceleration sensor, three-axis magnetic field sensor, and a three-axis gyro sensor with the range of magnetic field being ±1.3/1.9/2.5/4.0/4.7/5.6/8.1 gausses, the range of the acceleration being ±2g/±4g/±8g, and the range of the gyro being ±250/500/2000 dps. Abnormality can be identified by determining by monitoring and detecting a value of a body position according to the above-mentioned ranges. When the detected value exceeds the ranges, abnormal inclination is identified. When a variation of the inclination or body position exceeds a setting value or a threshold, the microcontroller issues, through a wireless signal transmitter (not shown), a signal.

For an example of the fabric layer 30 of the first and second embodiments of the detection module-included fabric according to the present invention (as shown in Figures 1 and 6), the fabric layer 30 comprises a signal transmission terminal 31 and the signal transmission terminal 31 may be coupled to at least one detection module 10 so that the variation of the physiological signal or the inclination angle of the human body detected by the detection module 10 can be transmitted to the signal transmission terminal 31. In addition, the signal transmission terminal 31 may be coupled to a signal transmitter (not shown) and the signal transmitter comprises a wireless transmission module that transmits, in a wireless manner, the variation of the physiological signal or the inclination angle of the human body to an electronic device (such as a smart mobile phone, a tablet computer, a notebook computer, a desktop computer, and medical facility) or to the cloud for realization of the detection result and to provide preventive medical treatment.

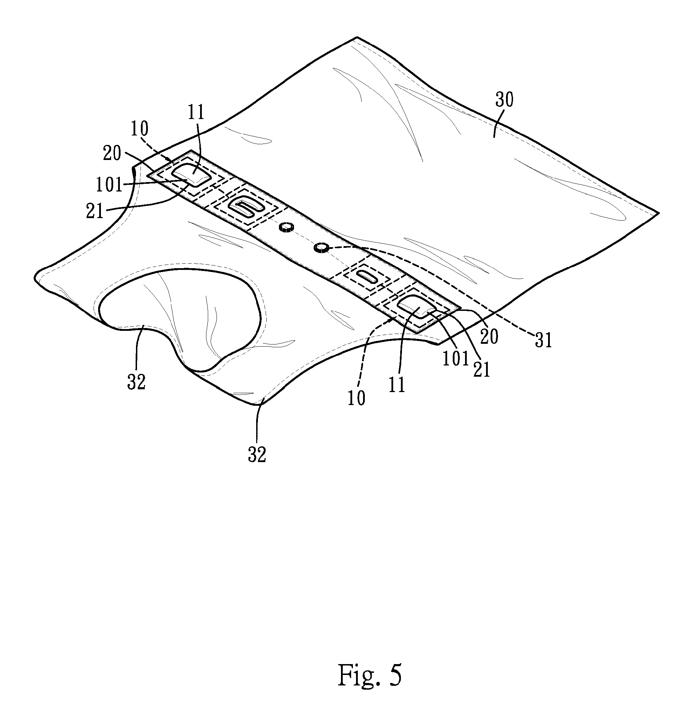

Further, in the first and second embodiments of the detection module-included fabric according to the present invention, an example of the site where the surface layer 20 and the fabric layer 30 are combined may be such that the surface layer 20 is cut to a desired shape that is one of for example a rectangle, a circle, an ellipse, and an irregular shape for being directly sewed to any desired portion of the fabric layer 30 (as shown in Figure 5). In sewing the surface layer 20 to the fabric layer 30, for edges of the surface layer 20 are all sewn or only two or three of the edges of the surface layer 20 are sewn in such a way that the contact surface 11 of the detection module 10 that is exposed through the window opening 21 of the surface layer 20 is positionable against and contactable with the surface of the human body. When the fabric layer 30 is shifted due to a movement of the user, since the contact surface 11 of the detection module 10 is positionable against the surface of the human body and shows an increased frictional force therebetween, during stretching or moving of the fabric layer 30, the contact surface 11 of the detection module 10 exhibits excellent attachment to the surface of the human body so that the contact engagement thereof with the surface of the human body is enhanced to make it hard for the detection module 10 that is mounted to the surface layer 20 to slide and thus, the detection module 10 is constantly kept in contact engagement with the surface of the human body to improve stability of signal detection and also improve the utilization of the device.

Further, in the first and second embodiments of the detection module-included fabric according to the present invention, the fabric layer 30 has an edge that may comprise at least one sewing thread 32 and an alternative site of combination of the surface layer 20 and the fabric layer 30 is sewing to the sewing thread 32 of the edge of the fabric layer 30 (as shown in Figures 1 and 6), where for the fabric layer 30 being tailored and sewn as a vest (or other types of clothing, such as a garment, trousers, gloves, an underwear, a corsage, and a tube-top, or the likes), the edge of the fabric layer 30 that is tailored and sewed or that is formed as a portion of the vest uses the sewing thread 32 for the sewing so that the edge of the vest that is formed of the fabric layer 30 may not get loosened for released of the thread and an aesthetic stitched edge may be presented. Thus, the surface layer 20 can be set at any desired site on the fabric layer 30 that is tailored and sewed as a vest, such as a shoulder and a chest. Taking the shoulder as an example, a width of the surface layer 20 between two opposite edges thereof may be greater than or equal to a width of the shoulder of the vest that is formed by tailoring and sewing the fabric layer 30 so that an edge of the surface layer 20 can be made adjacent to at least one sewing thread 32 of an edge of the shoulder of the vest formed by tailoring and sewing the fabric layer 30. The two edges of the surface layer 20 are then sewed to the sewing thread 32 of the edge of the shoulder of the vest formed by tailoring and sewing the fabric layer 30 so that the surface layer 20 is kept at a location corresponding to the shoulder of the vest formed by tailoring and sewing the fabric layer 30. Thus, the contact surface 11 of the detection module 10 that is exposed through the window opening 21 of the surface layer 20 can be positioned on and thus attached to the surface of the human body. When the fabric layer 30 is shifted due to a movement of the user, since the contact surface 11 of the detection module 10 is positionable against the surface of the human body and shows an increased frictional force therebetween, during stretching or moving of the fabric layer 30, the contact surface 11 of the detection module 10 exhibits excellent attachment to the surface of the human body so that the contact engagement thereof with the surface of the human body is enhanced to make it hard for the detection module 10 that is mounted to the surface layer 20 to slide and thus, the detection module 10 is constantly kept in contact engagement with the surface of the human body to improve stability of signal detection and also improve the utilization of the device.

As such, for both the first embodiment where the detection module 10 is arranged between the surface layer 20 and the fabric layer 30 and the second embodiment where the detection module 10 is arranged between the surface layer 20 and the protection layer 40, shifting of the detection module is difficult and thus, the detection module 10 is constantly kept in contact engagement with the surface of the human body to ensure stability of signal detection thereby.

Based on the above detailed description, those skilled in the art may appreciate that the present invention can achieve the above-discussed objectives. However, it is noted that the above description is made only to a preferred embodiment of the present invention and is not intending to limit the true scope where the present invention may be put into practice. Thus, simple and equivalent variations and modifications made on the disclosure of the specification and the attached claims are all considered within the scope of the present invention.

## Claims

1. A detection module-included fabric, comprising:
at least one detection module, which comprises a contact surface;
a surface layer, which is combined with the at least one detection module with the detection module located below the surface layer, the surface layer comprising at least one window opening, the contact surface of the detection module being exposed through the window opening of the surface layer; and
a fabric layer, which is combined with the surface layer, the fabric layer and the surface layer forming therebetween a gap, the detection module being located between the surface layer and the fabric layer.

2. The detection module-included fabric as claimed in Claim 1, wherein the detection module comprises a protection layer arranged therebelow, the protection layer having an edge combined with the surface layer, the protection layer being located between the detection module and the fabric layer.

3. The detection module-included fabric as claimed in Claim 2, wherein the protection layer comprises one of a piece of fabric and an insulation sheet.

4. The detection module-included fabric as claimed in Claim 1, wherein the fabric layer has an edge that comprises at least one sewing thread, the sewing thread of the fabric layer being coupled to an edge of the surface layer.

5. The detection module-included fabric as claimed in Claim 1, wherein the combination comprises one of sewing, ultrasonic fusion, hot fusion, and adhesive.

6. The detection module-included fabric as claimed in Claim 4, wherein the combination comprises one of sewing, ultrasonic fusion, hot fusion, and adhesive.

7. The detection module-included fabric as claimed in Claim 1, wherein the fabric layer comprises a signal transmission terminal, the signal transmission terminal being connected to the at least one detection module.

8. The detection module-included fabric as claimed in Claim 1, wherein the detection module comprises an electrode plate that is adapted to detect a physiological signal of a surface layer of a human body, the physiological signal comprising one of body temperature, heartbeat, and pulse.

9. The detection module-included fabric as claimed in Claim 2, wherein the detection module comprises an electrode plate that is adapted to detect a physiological signal of a surface layer of a human body, the physiological signal comprising one of body temperature, heartbeat, and pulse.

10. The detection module-included fabric as claimed in Claim 1, wherein the detection module comprises an inclination detection chip and a nicrocontroller, the microcontroller being connected to the inclination detection chip so that the microcontroller detects a variation of the inclination detection chip.

11. The detection module-included fabric as claimed in Claim 2, wherein the detection module comprises an inclination detection chip and a nicrocontroller, the microcontroller being connected to the inclination detection chip so that the microcontroller detects a variation of the inclination detection chip.

12. The detection module-included fabric as claimed in Claim 1, wherein the surface layer comprises one of a piece of fabric, an woven article, and a membrane.

13. The detection module-included fabric as claimed in Claim 2, wherein the surface layer comprises one of a piece of fabric, an woven article, and a membrane.

14. The detection module-included fabric as claimed in Claim 4, wherein the surface layer comprises one of a piece of fabric, an woven article, and a membrane.

15. The detection module-included fabric as claimed in Claim 1, wherein the fabric layer is tailored and sewn as one of a garment, trousers, gloves, underwear, a vest, a corsage, and a tube-top.

16. The detection module-included fabric as claimed in Claim 7, wherein the fabric layer is tailored and sewn as one of a garment, trousers, gloves, underwear, a vest, a corsage, and a tube-top.
